# EUROPEAN PATENT APPLICATION

(11) **EP 4 467 139 A1**
(43) Date of publication of application: **27.11.2024**
(21) Application number: 23382485.3
(22) Date of filing: 25.05.2023
(51) Int. Cl.: A61K 31/496, A61K 31/5025, A61K 31/506, A61P 7/06

(54) **ZAK ALPHA KINASE (MAP3K20) INHIBITORS FOR USE IN THE TREATMENT OF ANEMIA**

(71) Applicant: Universidad de Murcia, 30100 Murcia (ES); Consorcio Centro de Investigación Biomédica en Red, 28029 Madrid (ES); Fundación para la Formación e Investigación Sanitarias de la Región De Murcia (FFIS), 30120 El Palmar, Murcia (ES)
(72) Inventor: MULERO MÉNDEZ, Victoriano Francisco, 30100 Murcia (ES); RODRÍGUEZ RUIZ, Lola, 30100 Murcia (ES); LOZANO GIL, Juan Manuel, 30100 Murcia (ES); TYRKALSKA, Sylwia Dominika, 30100 Murcia (ES); CAYUELA FUENTES, María Luisa, 30120 Murcia (ES); GARCÍA MORENO, Diana, 30100 Murcia (ES); MARTÍNEZ LÓPEZ, Alicia, 30120 Murcia (ES)
(74) Representative: Hoffmann Eitle

(57) **Abstract**

The present invention refers to the medical field. Particularly, the present invention refers to ZAK alpha kinase (MAP3K20) inhibitors, or a composition comprising thereof, for use in the treatment of anemia.

## Description

### FIELD OF THE INVENTION

The present invention refers to the medical field. Particularly, the present invention refers to ZAK alpha kinase (MAP3K20) inhibitors, or a composition comprising thereof, for use in the treatment of anemia.

### STATE OF THE ART

### Anemia and its treatment

Anemia is a blood disorder in which the blood has a reduced ability to carry oxygen due to a lower than normal number of red blood cells, or a reduction in the amount of hemoglobin. Anemia is common in older people, and it becomes more so with advancing decades. Because the older population is increasing, the prevalence of anemia and consequently its impact on health and healthcare expenditure is expected to rise. Although the causes and consequences of anemia have not been fully elucidated and its etiology is occasionally elusive, clinical evidence has indicated that anemia itself is a cause of morbidity and it can complicate other health conditions. The clinical approach to anemia is evolving. In the past, anemia was mainly seen as a sign of underlying disease; today, anemia is considered to be a cause of severe deterioration of quality of life, morbidity, and decline in physical function, and a risk factor for death.

The treatment strategies for anemia include:
- Dietary supplements: they are used in the cases where anemia is caused by iron or B 12 deficiency.
- Blood transfusions: they are usually very safe because donated blood is carefully tested, handled, and stored. However, there is a small chance that the patient may have a mild to severe reaction to the donor blood. In addition, some people have health problems from getting too much iron from frequent transfusions. There is also a very small chance of getting an infectious disease.
- Blood and bone marrow transplant, also called hematopoietic stem cell transplant: it replaces faulty blood-forming stem cells with healthy cells. Blood or bone marrow transplants are usually performed in a hospital. Often, the patient must stay in the hospital for one to two weeks before the transplant to prepare. Patients may also receive special medicines and possibly radiation to destroy abnormal stem cells and to weaken their immune system so that it won't reject the donor cells after the transplant.

- Surgery may be needed to stop internal bleeding.
- Erythropoiesis-stimulating agents: they are used to treat anemia caused by chronic kidney failure, some anticancer drugs, and certain treatments for HIV. They may also be used to lower the number of blood transfusions needed during and after certain major surgeries.

### The novel role of the inflammasome in hematopoiesis

Inflammasomes are cytosolic pattern recognition receptors (PRRs) that detect pathogen- and danger-associated molecular patterns (PAMPs and DAMPs). The relevance of inflammasomes in human biology is highlighted by their involvement in numerous inflammatory disorders. Inflammasome assembly is a complex process and involves the activation of a sensor, which belongs to the NOD-like receptor (NLR) or absent of melanoma 2-like receptor (AIM2) family, recruitment and polymerization of the adaptor Apoptosis-associated speck-like protein containing a CARD (ASC), and activation of the effector caspase-1 (CASP1) or CASP4/CASP5 (CASP11 in mice).

The NLR family pyrin domain containing 1 (NLRP1) inflammasome was the first inflammasome identified more than 20 years ago. However, its activation mechanism has remained obscure, probably due to the presence of different NLPR1 paralogs in mice, and their obvious differences with human NLRP1 in structure, ASC requirement and activation mechanisms. NLPR1 is highly expressed in the skin and, in fact, gain-of-function mutations result in inflammatory skin diseases and cancer susceptibility syndromes primarily driven by hyperproduction of interleukin-1β (IL-1β). Similarly, loss-of-function mutations of its direct inhibitor Dipeptidyl Peptidase 9 (DPP9) leads to a lethal autoinflammatory disorder characterized by hyperproduction of IL-1β. Recent studies revealed two physiological activators of human NLRP1: (i) viral 3C proteases that cleave its N terminus, and their mode of action was in line with the functional degradation reported for mice NLRP1B, and (ii) double-stranded RNA (dsRNA). Notably, dsRNA did not activate mouse NLRP1B. More recently, two independent laboratories demonstrated that human, but not mouse, NLRP1 is activated by direct phosphorylation of its linker region by MAK kinase P38 (MAPK14), which is activated by the ZAKα isoform of MAP3K20 in response to either ribotoxic stress response to UV or alphavirus. This activation model also involved ubiquitination and N terminal degradation of NLRP1 but being independent of DPP9.

It was recently shown that the canonical inflammasome is activated in hematopoietic stem and progenitor cells (HSPCs) and regulates erythroid-myeloid lineage decision by cleaving the erythroid transcription factor GATA binding protein 1 (GATA1). Although this mechanism is conserved in zebrafish and human, the specific NLR sensor involved, and its activation mechanism remained unknown.

There is an unmet medical need to develop therapeutic strategies for the treatment of anemia. In particular, there is a need to develop erythropoiesis-stimulating agents that may minimize the use of transfusions. The present invention is focused on solving this problem and a novel therapeutic strategy for the treatment of anemia is herein provided.

### DESCRIPTION OF THE INVENTION

### Brief description of the invention

As explained above, the present invention refers to ZAKα kinase (MAP3K20) inhibitors, or a composition comprising thereof, for use in the treatment of anemia.

Given that cellular stress was recently found to activate ZAKα resulting in the subsequent phosphorylation and activation of the human NLRP1 inflammasome, the inventors of the present invention hypothesised that a similar mechanism could operate to regulate haematopoiesis.

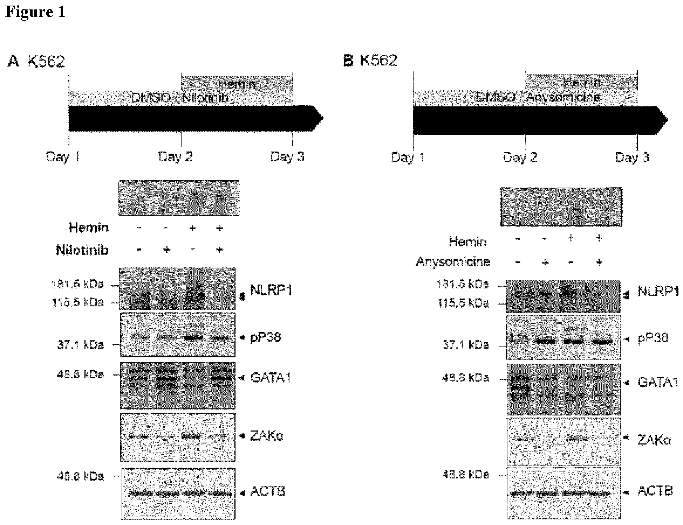

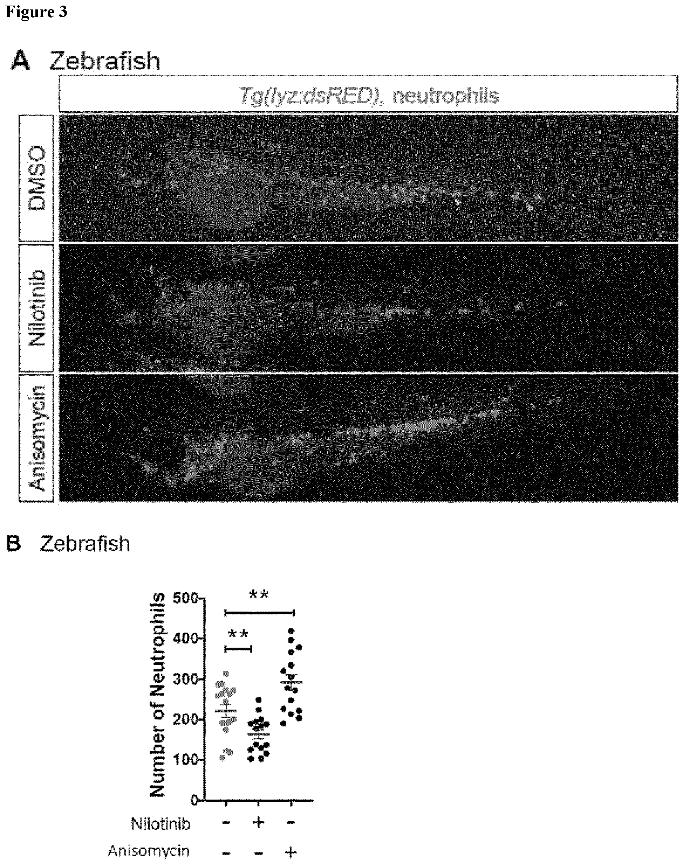

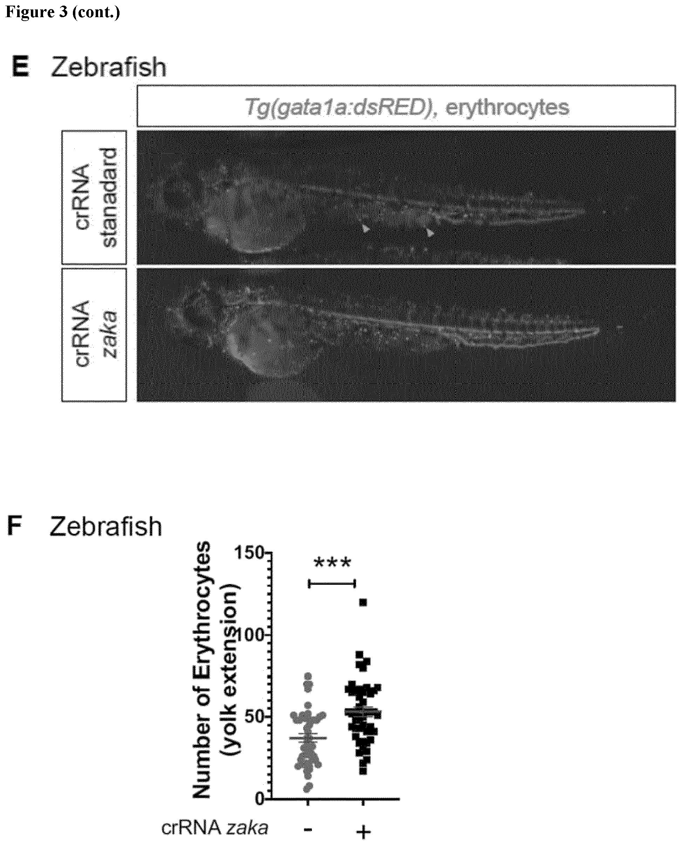

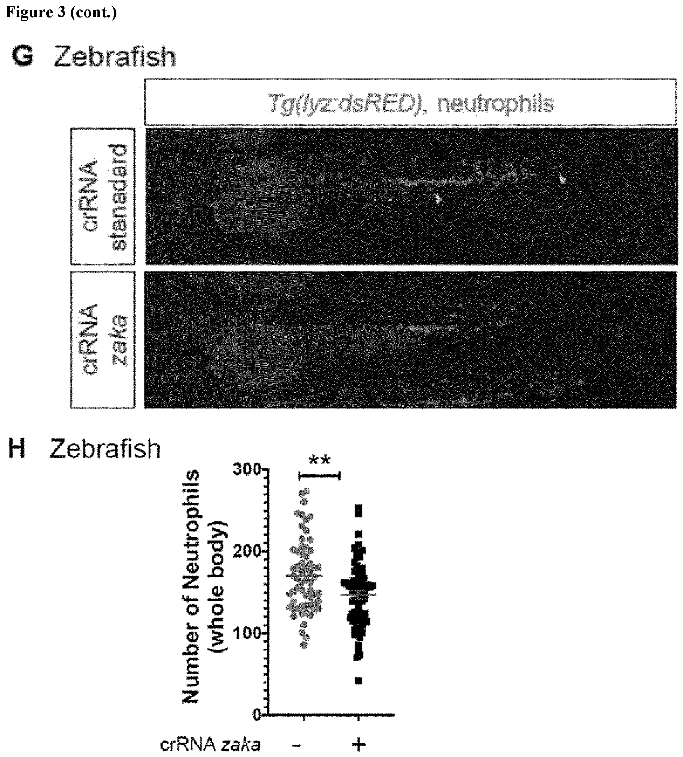

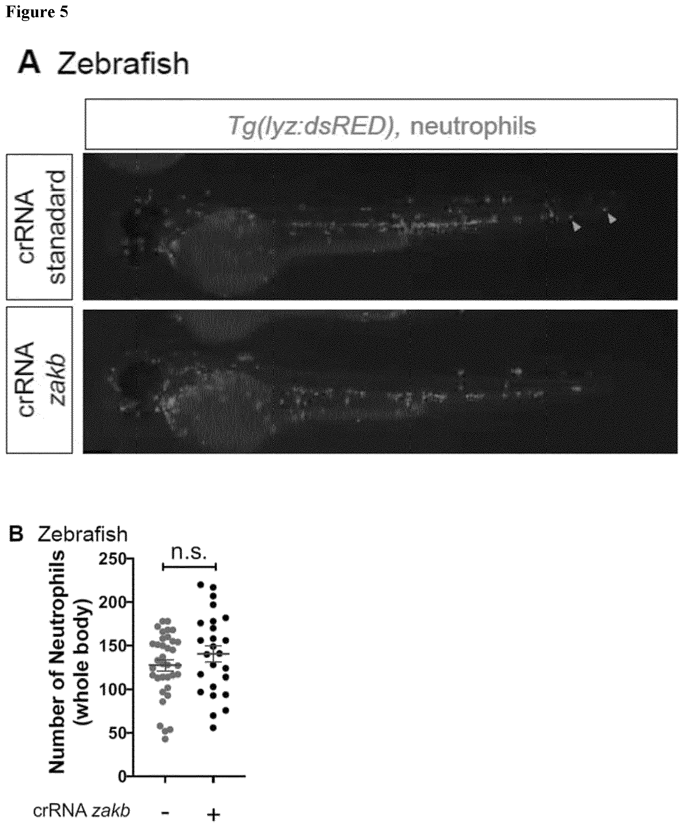

To test this hypothesis, the inventors of the present invention quantified the abundance of proteins involved in the ZAKα/P38 signalling pathway in K562 cells upon hemin-induced erythroid differentiation. Hemin induction resulted in an increase in ZAKα, phosphorylated P38, and NLRP1 **(****Figure 1A****),** indicating that the NLRP1 inflammasome as well as the ZAKα/P38 signalling pathway were indeed involved in the regulation of haematopoiesis. Additional experiments performed in zebrafish further supported this idea:
- Nlrp1 (NLRP1 zebrafish homologue) deficiency led to a reduction in the total abundance of neutrophils in the body **(****Figure 5B****).**
- Zaka (ZAKα zebrafish homologue) deficiency reduced the abundance of neutrophils **(****Figure 3G and 3H****),** while promoting erythropoiesis **(****Figure 3E and 3F****).**

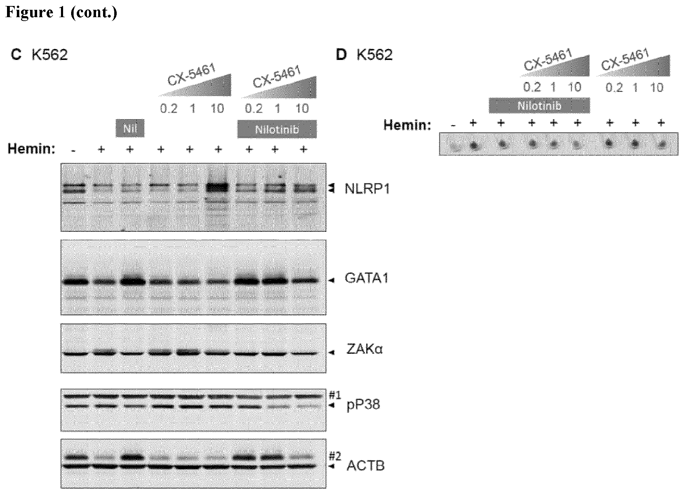

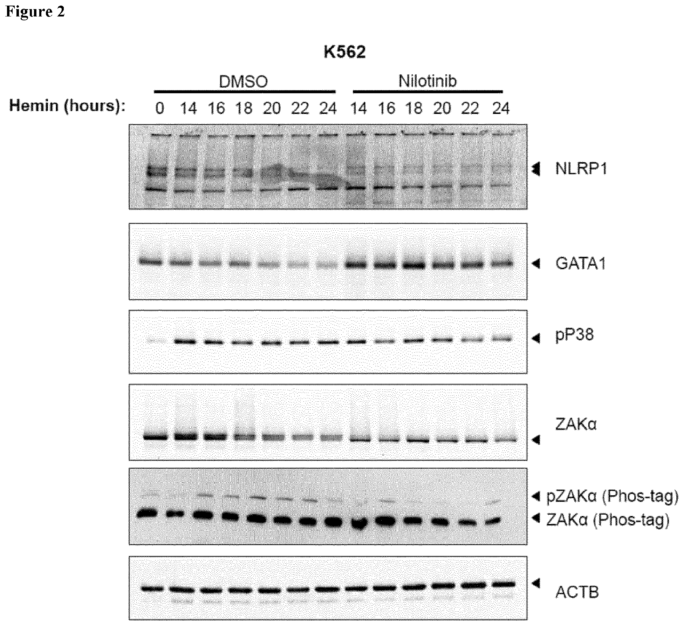

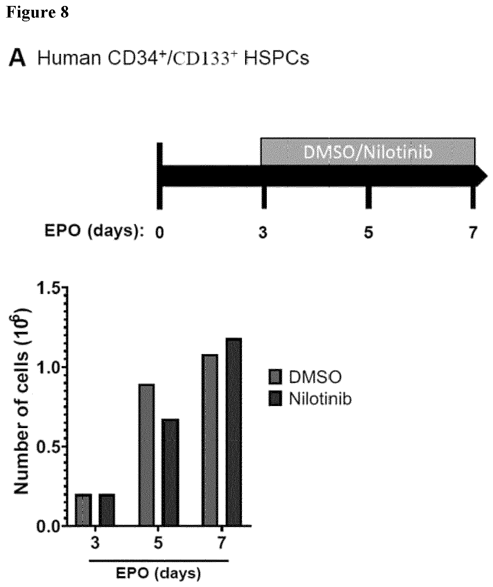

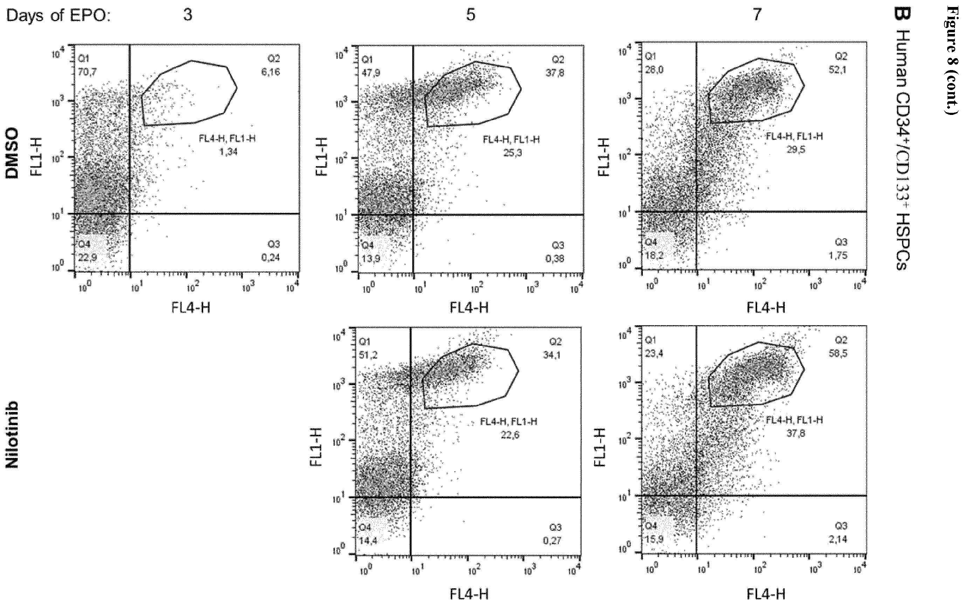

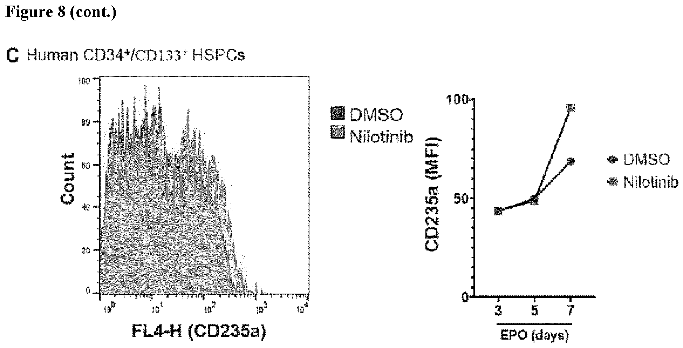

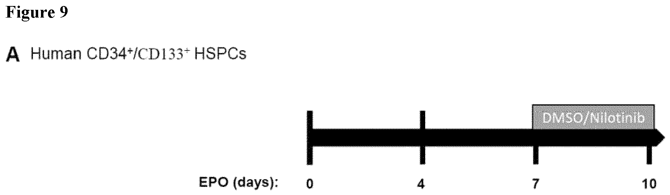

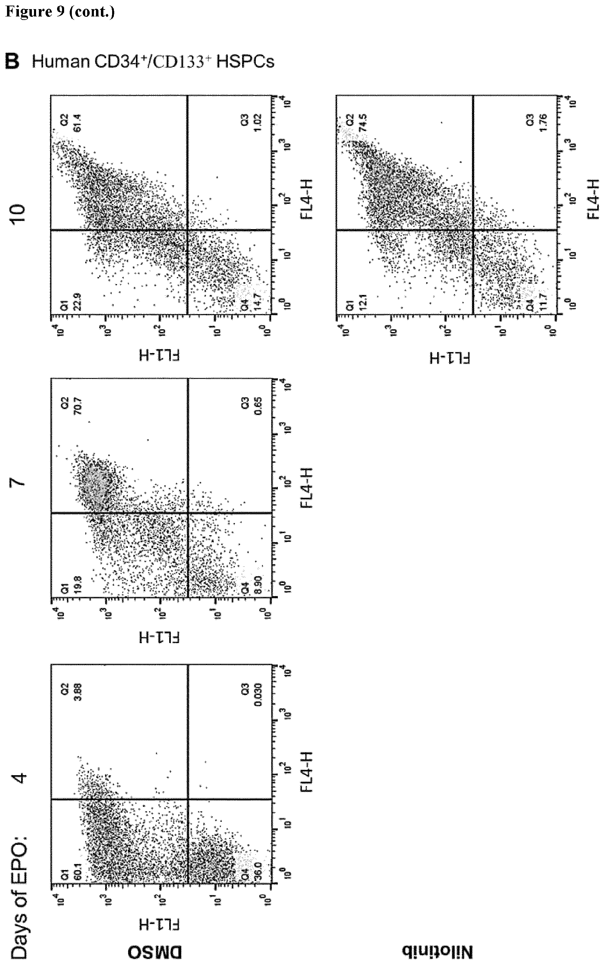

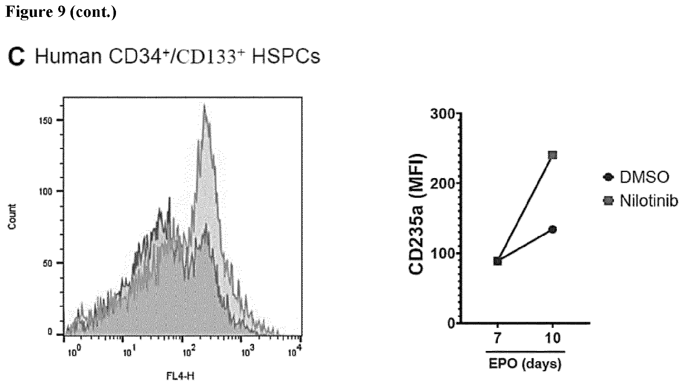

In this context, the inventors hypothesised that altering this signalling pathway by using ZAKα kinase inhibitors, could have an effect in the regulation of hematopoiesis in K562 cells. As a proof of concept, they evaluated the effect of nilotinib, a kinase inhibitor that has a high affinity for ZAKα. Among others, nilotinib administration was found to:
- Promote erythropoiesis in K562 cells, as reflected by an increase in haemoglobin accumulation and by an increased expression of GATA1, the master regulatory factor of erythropoiesis **(****Figure 1A** **and** **2****).**
- Alter the abundance of ZAK alpha, phosphorylated ZAKα and P38, and NLRP1, both in basal conditions and upon hemin-induced erythroid differentiation **(****Figure 1A****,** **1C** **and** **2****),** and upon anisomycin, administration, an antibiotic that promotes ribotoxic stress-mediated activation of NLRP1 **(****Figure 1B****).**
- Induce the expression of GATA1, even in the presence of CX-5461 **(****Figure 1C****),** an RNA polymerase I inhibitor that impairs erythroid differentiation in K562 cells **(****Figure 1D****).**
- Promote erythropoiesis in zebrafish **(****Figure 3E and 3F****),** while reducing neutrophilia **(****Figure 3A-3D****).**
- Nilotinib accelerated erythropoiesis of primary HSPCs from healthy donors **(****Figures 8** **and** **9****)**

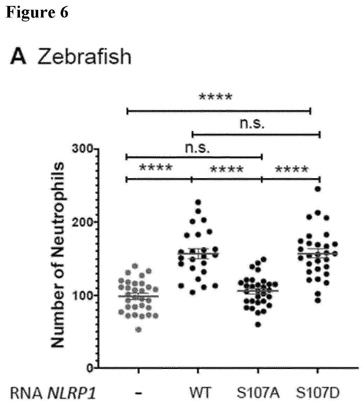

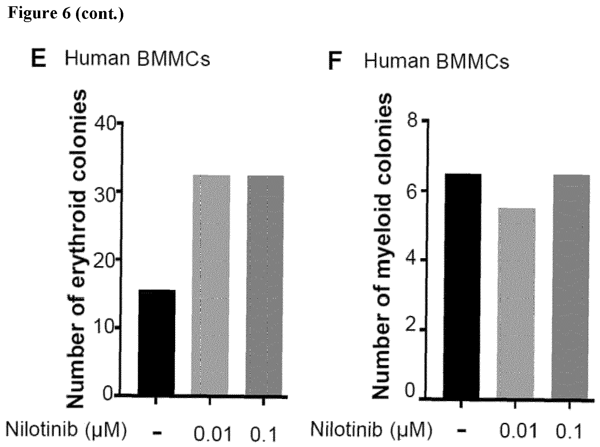

These results indicate, using nilotinib as a proof of concept, that ZAKα kinase inhibitors promote myelopoiesis and erythroid differentiation. In this context, the inventors hypothesised that ZAKα kinase inhibitors may be effective as a treatment against anemia, a condition characterized by a pathologically low abundance of erythrocytes. Thus, the inventors moved on to evaluate the effect of ZAKα kinase inhibitors in anemia, using Diamond Blackfan anemia (DBA) as a proof of concept. Nilotinib increased the abundance of erythroid colonies derived from bone marrow mononuclear cells of a DBA patient **(****Figure 6E****),** while no effect was observed in the number of myeloid colonies **(****Figure 6F****).**

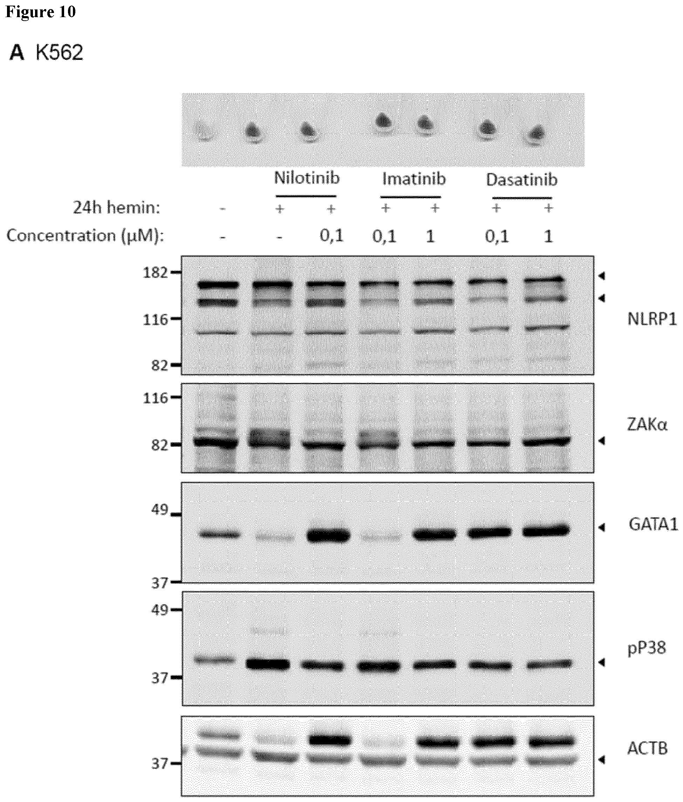

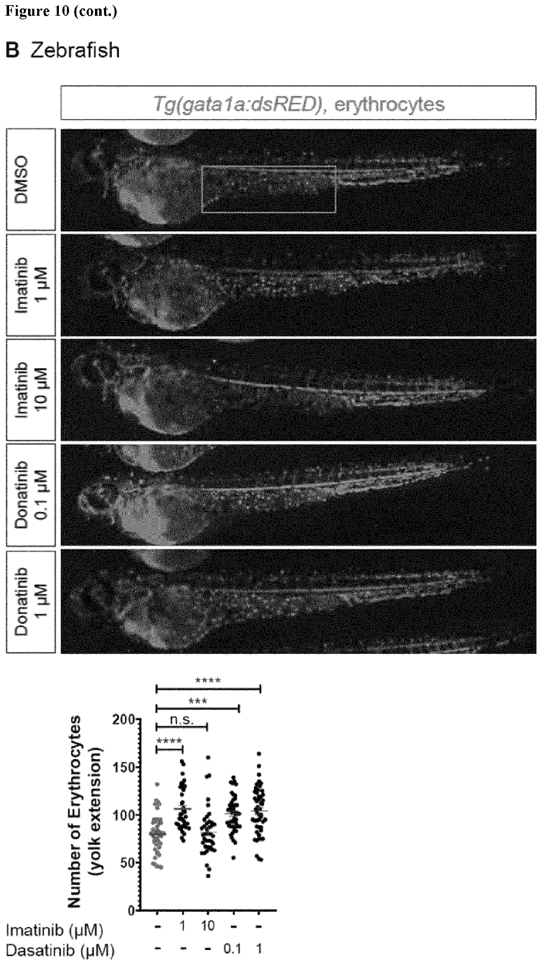

To further support the role of ZAKα kinase inhibitors in the promotion of erythroid differentiation, two tyrosine kinase inhibitors that are approved by the FDA/EMA with the same indications as nilotinib. Both imatinib and dasatinib phenocopied the effects of nilotinib in both human K562 cells and zebrafish larvae: they promoted erythroid differentiation of K562 cells by blocking the ZAKα/P38 axis and enhancing GATA1 amount **(****Figure 10A****)** and increased the number of erythrocytes in zebrafish larvae **(****Figure 10B****).**

Together these results demonstrate the role of ZAKα kinase inhibitors in the promotion of erythropoiesis. These results also indicate, using DBA as a proof of concept, that these inhibitors can be used for the treatment of anemia.

This invention reports that inhibition of the ZAKα/P38 axis with the FDA/EMA-approved drugs nilotinib, dasatinib and imatinib promotes myelopoiesis and erythropoiesis in zebrafish and human, showing the clinical relevance of these findings to treating anemia, particularly congenital anemia or anemia of inflammation.

So, the first embodiment ZAK alpha kinase inhibitors for use in the treatment of anemia.

The second embodiment of the present invention refers to a composition comprising ZAK alpha kinase inhibitors and, optionally, pharmaceutically acceptable excipients or carriers, for use in the treatment of anemia.

The third embodiment of the present invention refers to an *in vitro* method for screening, identifying and/or producing compounds for use in the treatment of anemia which comprises: a) Assessing ZAK alpha kinase enzyme activity once the candidate compound has been incubated with ZAK alpha kinase enzyme, and b) wherein if an inhibition of ZAK alpha kinase enzyme activity is observed, it is indicative that the candidate compound may be effective in the treatment of anemia.

In a preferred embodiment, the ZAK alpha kinase inhibitors are used in the treatment of congenital anemia or anemia of inflammation.

In a preferred embodiment the composition is used in the treatment of congenital anemia or anemia of inflammation.

In a preferred embodiment, the congenital anemia is selected from the group comprising: Diamond-Blackfan anemia, Fanconi anemia and/or y thalassemia.

In a preferred embodiment, the anemia of inflammation is a non-ferropenic acquired anemia.

In a preferred embodiment, the inhibitor is a tyrosine kinase inhibitor.

In a preferred embodiment, the inhibitor is a tyrosine kinase inhibitor selected from the group comprising: Nilotinib, Dasatibin, Imatinib, Ponatinib and/or Bosutinib.

In a preferred embodiment, the ZAK alpha kinase inhibitors selected from the group comprising: Nilotinib, Dasatibin, Imatinib, Ponatinib and/or Bosutinib, are used in the treatment of congenital anemia.

In a preferred embodiment, the composition is administered by enteral or parenteral administration, preferably oral or intravenous administration.

Alternatively, the present invention also refers to a method for treating anemia diseases which comprises the administration of a therapeutically effective dose or amount of any of the ZAK alpha kinase inhibitors described above, or compositions comprising thereof. In a preferred embodiment the composition is used in the treatment of congenital anemia or anemia of inflammation. In a preferred embodiment, the congenital anemia is selected from the group comprising: Diamond-Blackfan anemia, Fanconi anemia and/or y thalassemia. In a preferred embodiment, the anemia of inflammation is a non-ferropenic acquired anemia.

In the context of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of'. Thus, the phrase "consisting of" indicates that the listed elements are required or mandatory, and that no other elements may be present.
- "Pharmaceutically acceptable excipient or carrier" refers to an excipient that may optionally be included with the pharmaceutical composition of the invention and that causes no significant adverse toxicological effects to the patient.
- By "therapeutically effective dose or amount" of the pharmaceutical composition of the invention is intended an amount that, when administered as described herein, brings about a positive therapeutic response in a subject having cancer. The exact amount required will vary from subject to subject, depending on the age, and general condition of the subject, the severity of the condition being treated, mode of administration, and the like. An appropriate "effective" amount in any individual case may be determined by one of ordinary skill in the art using routine experimentation, based upon the information provided herein.

### Description of the figures

**Figure 1****. ZAKα/P38 signaling pathway is activated in K562 after erythroid differentiation and** its **inhibition facilitates terminal erythroid differentiation.** K562 cells were pre-treated with 0.1 µM nilotinib (Nil) **(A, C, D),** 1 µM anisomycin **(B)** and/or the indicated concentrations of the RNA Pol II inhibitor CX-5461 for 24 h, and then differentiated with 50 µM hemin for another 24 h. Hemoglobin accumulation **(A, B, D)** and NLRP1, phosphorylated P38, GATA1, ZAKα and ACTB **(A- C)** amounts were then evaluated by western blot. Immunoblots are representative of 3 independent experiments. #1, ACTB; #2, GATA1.
**Figure 2****. The ZAKα/P38 signaling pathways is activated in K562 after erythroid differentiation.** K562 cells were pre-treated with 0.1 µM nilotinib for 24 h and then differentiated with 50 µM hemin for the indicated times. The amounts of NLRP1, GATA1, phosphorylated P38, total ZAKα, phosphorylated ZAKα (Phos-tag) and ACTB were then evaluated by western blot.

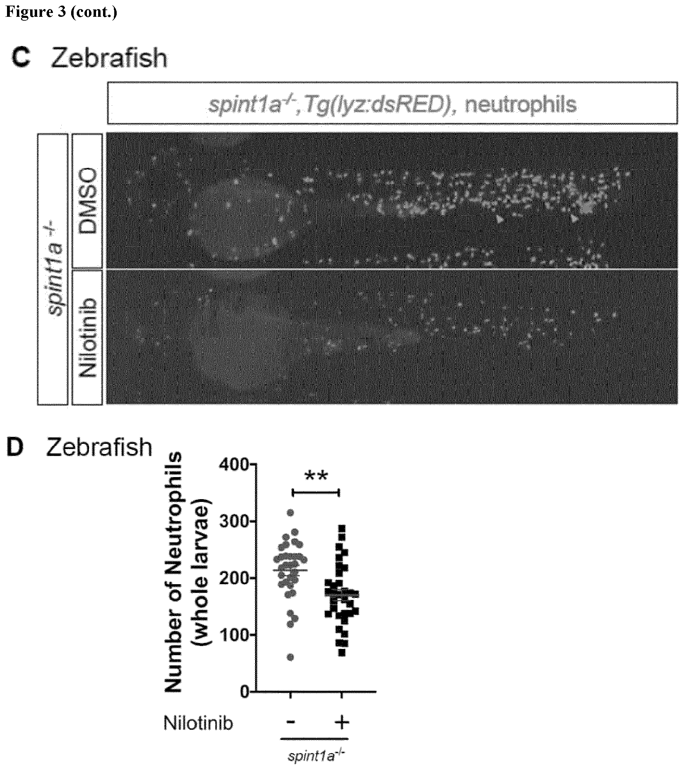

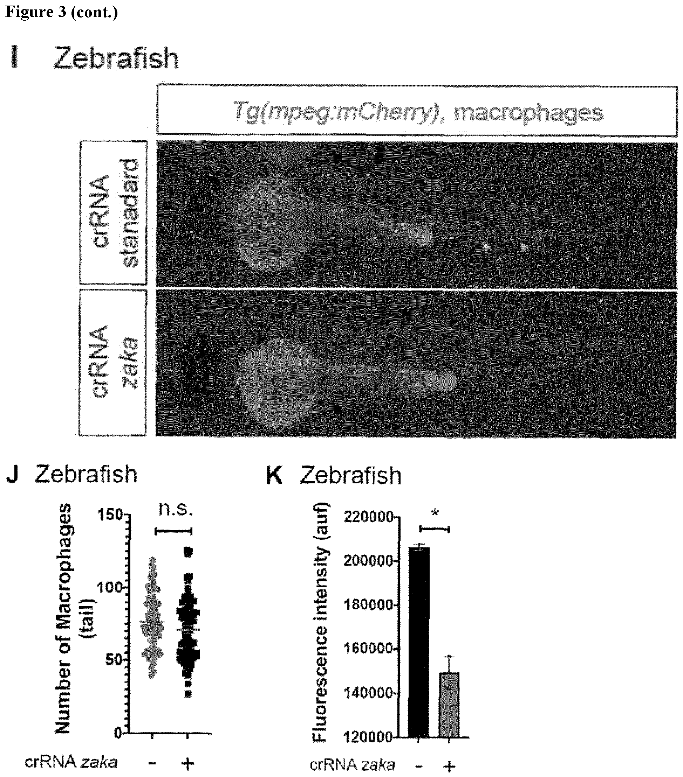

**Figure 3****. ZAKα regulates hematopoiesis in zebrafish through the Nlrp1 inflammasome.** Number of neutrophils **(B, D, H),** erythrocytes **(F)** and macrophages **(J),** and caspase-1 activity **(K)** in 2 dpf larvae either treated from 1 to 2 dpf by bath immersion with 1 µM nilotinib and/or 100 µM anisomycin **(A-D)** or obtained by injecting one-cell stage embryos with standard or *zaka* crRNAs/Cas9 complexes **(E-K).** Representative images of neutrophils **(A, C, G),** erythrocytes **(E)** and macrophages **(I)** are also shown. Each dot represents one individual and the mean ± SEM for each group is also shown. P values were calculated using one-way ANOVA and Tukey's multiple range test **(B)** or Student's t-test **(D, F, H, J, K).** n.s., non-significant; ^{∗}P<0.05; ^{∗∗}P<0.01; ^{∗∗∗}P<0.001.

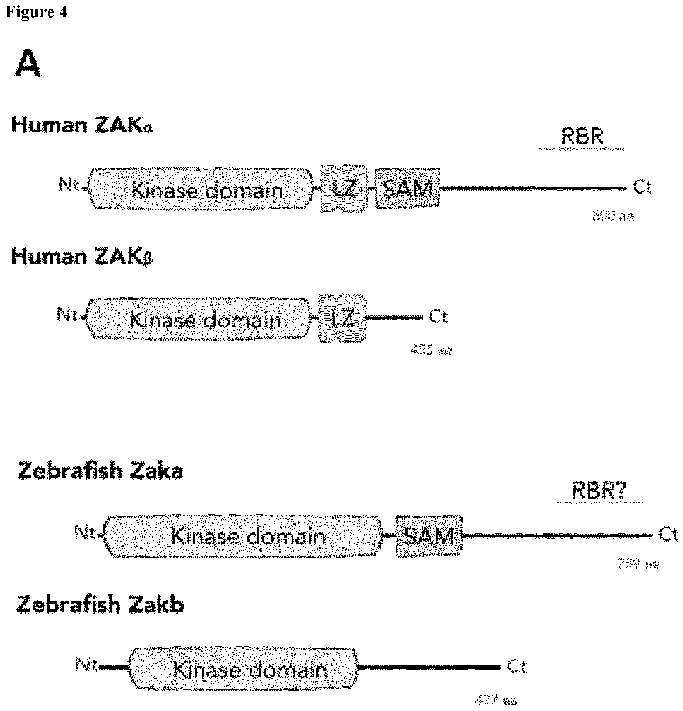

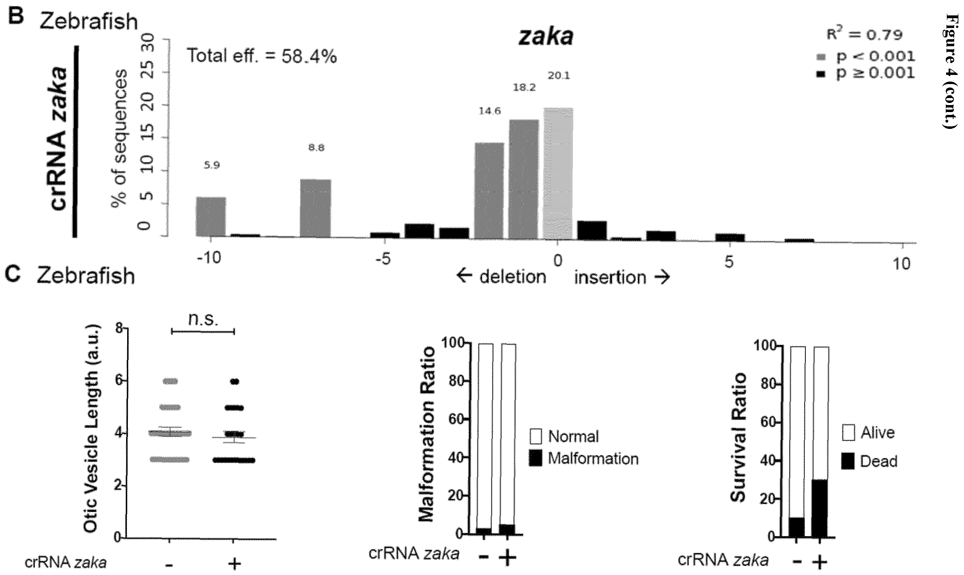

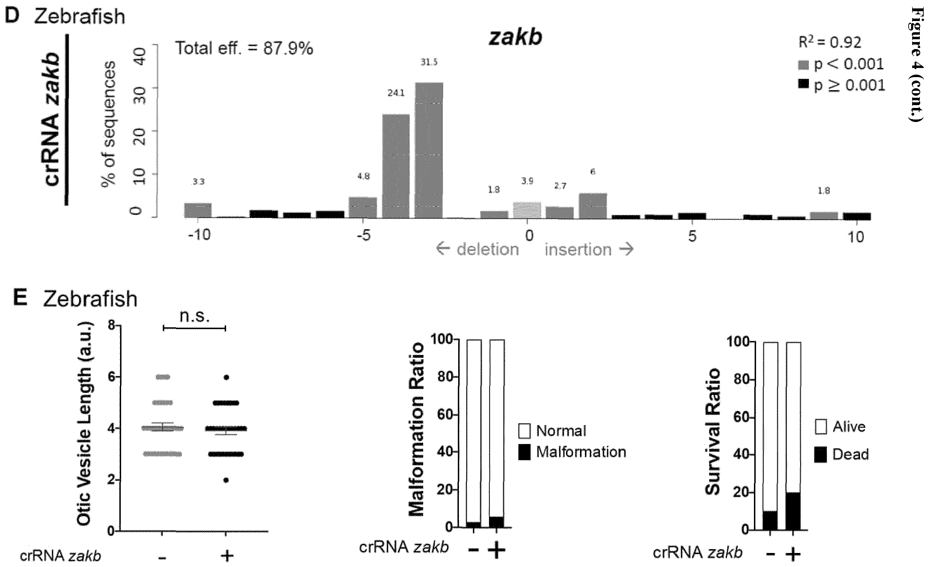

**Figure 4****. Comparison of human and zebrafish ZAKα and ZAKβ. (A)** Schemes showing domain organization of human and zebrafish ZAKα and ZAKβ. **(B, D)** Analysis of genome editing efficiency in larvae injected with either *zaka* **(B)** or *zakb* **(D)** crRNA/Cas 9 complexes and quantification rate of nonhomologous end joining mediated repair showing all insertions and deletions at the target site using TIDE (https://tide.nki.nl). **(C, E)** Developmental stage, malformation, and survival of Zaka- **(C)** and Zakb-deficient **(E)** embryos were determined at 24 hpf. Each dot represents one individual and the mean ± SEM for each group is also shown. P values were calculated by Student's *t* test. n.s., non-significant. a.u., arbitrary units.

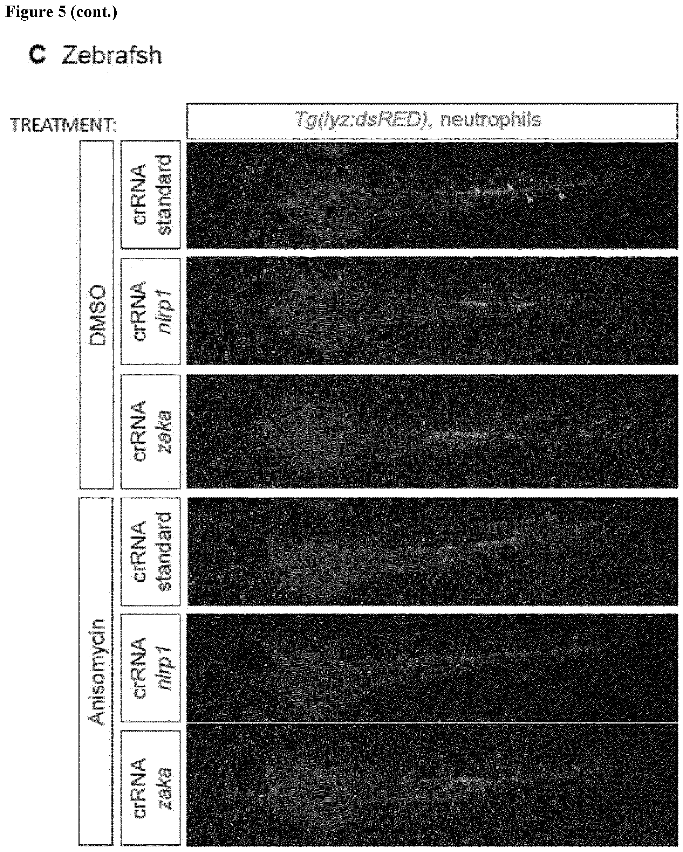

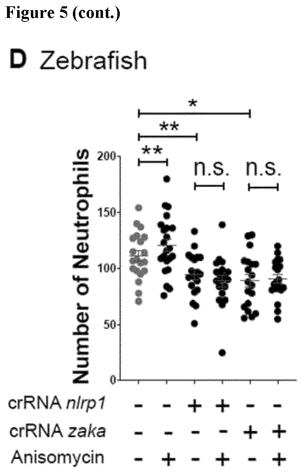

**Figure 5****. Zaka mediates the activation of the Nlrp1 inflammasome to regulate zebrafish hematopoiesis.** Representative images and number of neutrophils in Zakb- **(A, B),** Zaka- **(C, D)** and Nlrp 1-deficient **(C, D)** larvae of 2 dpf obtained by injecting one-cell stage embryos with standard, *zakb* and *zaka* crRNAs/Cas9 complexes. Fluorescent cells in each reporter line are indicated with arrowheads. Each dot represents one individual and the mean ± SEM for each group is also shown. P values were calculated by one-way ANOVA and Tukey's multiple range test. n.s., non-significant. ^{∗}P<0.05; ^{∗∗}P<0.01; ^{∗∗∗∗}P<0.0001.

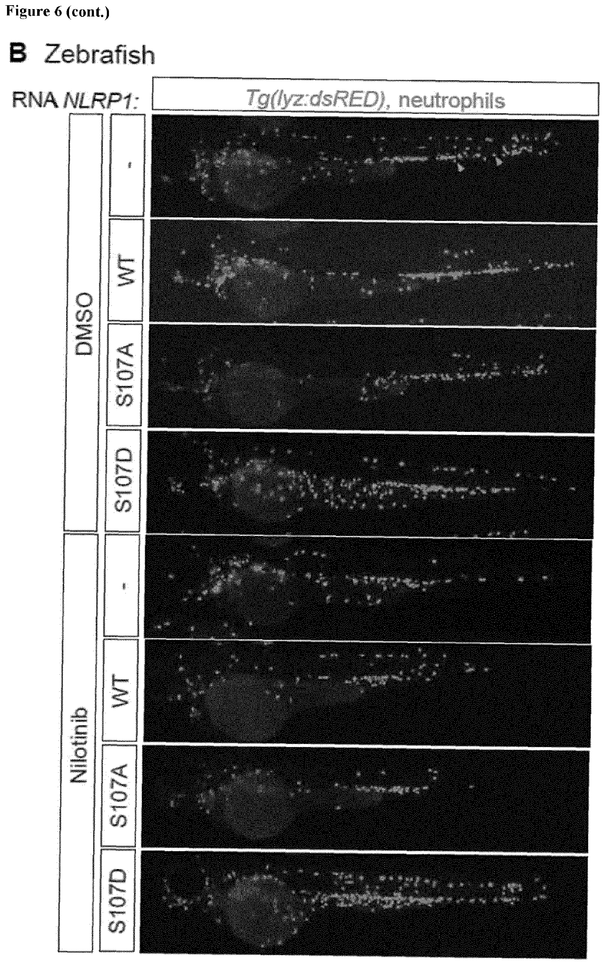

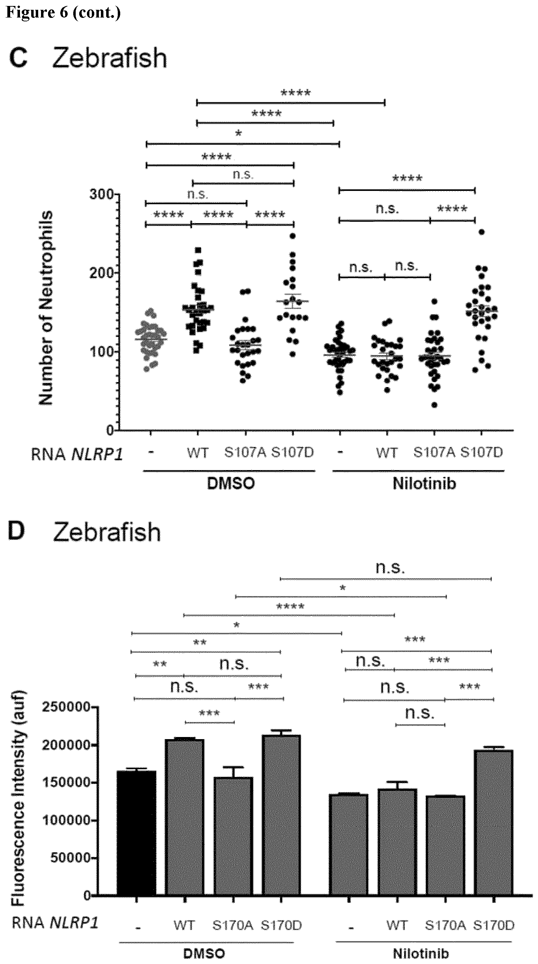

**Figure 6****. NLRP1 is activated by phosphorylation of the linker domain after activation of ZAKα and pharmacological inhibition of this signaling pathway promotes erythropoiesis in DBA. (A-D)** Number of neutrophils **(A-C),** and caspase-1 activity **(D)** in 2 dpf larvae obtained by injection of one-cell stage embryos with human NLRP1 (wild type-WT, S107A and S107D) and treated from 1 to 2 dpf by bath immersion with 1 µM nilotinib **(B-D).** Representative images of neutrophils (arrows) are also shown in C. **(E, F)** BMMCs from a DBA patient were incubated for 2 weeks in human methylcellulose complete medium at 37 °C in the presence or absence of the indicated concentrations of nilotinib, and colonies were counted using standard morphological criteria. Each dot represents one individual and the mean ± SEM for each group is also shown. P values were calculated by one-way ANOVA and Tukey's multiple range test. n.s., non-significant; ^{∗}P<0.05; ^{∗∗}P<0.01; ^{∗∗∗}P<0.001; ^{∗∗∗∗}P<0.0001.

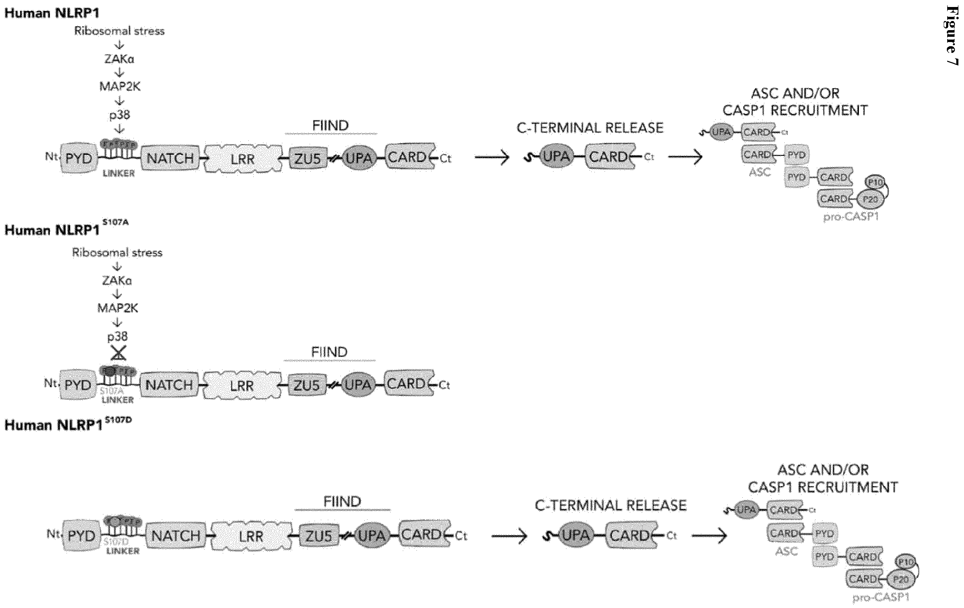

**Figure 7****. Activation of NLRP1 by the ZAKα/P38 signaling pathway.** Schemes showing the domain organization of human wild type NLRP1 and the S107A S107D mutants. Phosphorylation of the linker domain by ZAKα (MAP3K)/MAP2K/P38 (MAPK) axis is highlighted. Note that S107A cannot be phosphorylated by P38, while phosphomimetic S107D is constitutively active independently of P38 activation.
**Figure 8****. Nilotinib promotes erythropoiesis of primary CD34⁺/CD133⁺ HSPCs from healthy donors. (A)** Primary human CD34⁺/CD133⁺ cells were differentiated with erythropoietin (EPO) and 0.1 µM nilotinib was added from 3 to 7 d of culture and the number of life cells calculated by the trypan blue exclusion assay. **(B, C)** Cells were stained with anti-CD235a-APC and anti-CD71-FITC, and erythroid differentiation was then analyzed by flow cytometry. Representative dot plots at different differentiation times **(B)** and overlapped histogram of CD235a at 7 d and the mean fluorescence intensity (MFI) at different differentiation times **(C)** are shown.
**Figure 9****. Nilotinib promotes erythropoiesis of primary CD34⁺/CD133⁺ HSPCs from healthy donors. (A)** Primary human CD34⁺/CD133⁺ cells were differentiated with EPO and 0.1 µM nilotinib was added from 7 to 10 d of culture and the number of life cells calculated by the trypan blue exclusion assay. **(B, C)** Cells were stained with anti-CD235a-APC and anti-CD71-FITC, and erythroid differentiation was then analyzed by flow cytometry. Representative dot plots at different differentiation times **(B)** and overlapped histogram of CD235a at 10 d and the mean fluorescence intensity (MFI) at different differentiation times **(C)** are shown.
**Figure 10****. Imatinib and dasatinib promote erythropoiesis in human and zebrafish. (A)** K562 cells were pretreated with the indicated concentrations of nilotinib, imatinib or dasatinib for 24 h and then differentiated with 50 µM hemin for 24 h. The amounts of NLRP1, GATA1, phosphorylated P38, total ZAKα and ACTB were then evaluated by western blot. **(B)** Zebrafish larvae were treated from 1 to 2 dpf with the indicated concentrations of nilotinib, imatinib or dasatinib. Representative images and number of neutrophils in larvae of 2 dpf treated by bath immersion with the indicated concentrations of imatinib and dasatinib. Each dot represents one individual and the mean ± SEM for each group is also shown. P values were calculated by one-way ANOVA and Tukey's multiple range test. n.s., non-significant. ^{∗∗∗}P<0.001; ^{∗∗∗∗}P<0.0001.

### Detailed description of the invention

The present invention is illustrated by means of the Examples set below without the intention of limiting its scope of protection.

### Example 1. Material and Methods

### Example 1.1. Zebrafish

Zebrafish (*Danio rerio* H.) were obtained from the Zebrafish International Resource Center and mated, staged, raised and processed as described in The Zebarfish Book. The lines *Tg(mpx:eGFP)ⁱ¹¹⁴, Tg(lyz:DsRED2)^{nz50}, Tg(mfap4:mCherry)^{ump6}, Tg(gatala:DsRed)^{sd2}, Tg(runxl:GAL4)^{utn6}* ), *Tg(UAS-E1B:NTR-mCherry)* and casper (*mitfa*^{*w2*/}*^{w2}; mpv17*^{*a9*/}*^{a9})* were previously described. *spint1a*^{*hi2217Tg*/*hi2217Tg*} was isolated from insertional mutagenesis screens. The experiments performed comply with the Guidelines of the European Union Council (Directive 2010/63/EU) and the Spanish RD 53/2013. The experiments and procedures performed were approved by the Bioethical Committees of the University of Murcia (approval number #669/2020).

*CRlSPR and RNA injections in zebrafish.* Negative control crRNA (catalog no. 1072544, crSTD) and crRNA for *nlrpl, zaka* and *zakb* **(Table 1),** and tracrRNA were resuspended in Nuclease-Free Duplex Buffer to 100 µM. One µl of each was mixed and incubated for 5 min at 95 °C for duplexing. After removal from heat and cooling to room temperature, 1.43 µl of Nuclease-Free Duplex Buffer was added to the duplex, yielding a final concentration of 1000 ng/µl. Finally, the injection mix was prepared by mixing 1 µl of duplex, 2.55 µl of Nuclease-Free Duplex Buffer, 0.25 µl Cas9 Nuclease V3 (IDT, 1081058) and 0.25 µl of phenol red, giving final concentrations of 250 ng/µl of gRNA duplex and 500 ng/µl of Cas9. The prepared mix was microinjected into the yolk sac of one- to eight-cell-stage embryos using a microinjector (Narishige) (0.5-1 nl per embryo). The same amounts of gRNA were used in all experimental groups. The efficiency of each crRNA was tested by amplifying the target sequence with a specific pair of primers **(Table** 2) and the amplicon was then analyzed the TIDE webtool (https://tide.nki.nl/).

Human *NLRP1-FLAG* (accession number NM_033004.4) (wild type, S107A and S107D) were synthesized by GeneScript. *In vitro*-transcribed RNA was obtained following the manufacturer's instructions (mMESSAGE mMACHINE kit, Ambion). RNA was mixed with microinjection buffer and microinjected into the yolk sac of one-cell-stage embryos using a microinjector (Narishige; 0.5-1 nl per embryo). The same amount of RNA was used for all experimental groups.

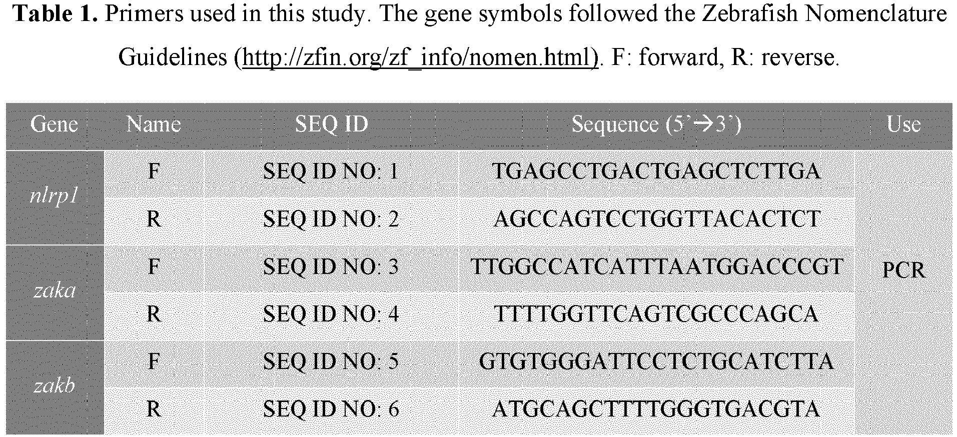

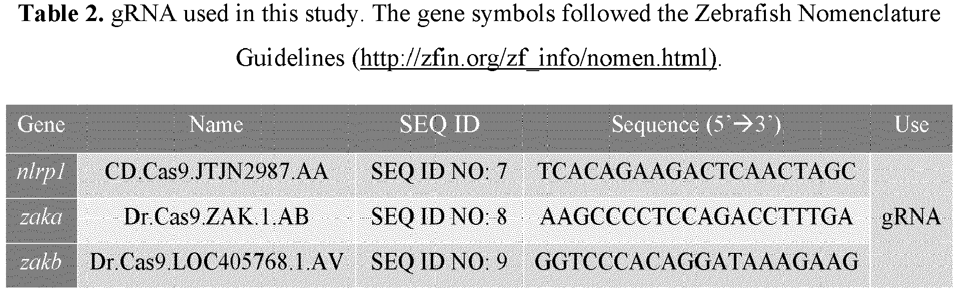

### Example 1.2. Chemical treatments of zebrafish larvae

One dpf larvae were manually dechorionated and treated for 24 h at 28 °C by bath immersion with the tyrosine kinase inhibitor nilotinib (AMN107, 1 µM), imatinib (STI571, 1 and 10 µM), dasatinib (BMS-354825, 0.1 and 1 µM) and the protein synthesis inhibitor anisomycin (100 µM) (all from MedChemExpress) diluted in egg water supplemented with 0.1% DMSO.

### Example 1.3. Caspase-1 activity assays

Caspase-1 activity was determined with the fluorometric substrate Z-YVAD 7-Amido-4-trifluoromethylcoumarin (Z-YVAD-AFC, caspase-1 substrate VI, Calbiochem). Briefly, 25-35 larvae were lysed in hypotonic cell lysis buffer on ice for 10 min. For each reaction, 100 µg of protein were incubated for 90 min at room temperature with 50 mM YVAD-AFC and 50 µl of reaction buffer. After incubation, the fluorescence of AFC released from the Z-YVAD-AFC substrate was measured with a FLUOstart spectrofluorometer (BGM, LabTechnologies) at an excitation wavelength of 405 nm and an emission wavelength of 492 nm. A representative graph of caspase-1 activity of three replicates is shown in the figures.

### Example 1.4. Imaging of zebrafish larvae

Larvae were anaesthetized in embryo medium with 0.16 mg/ml buffered tricaine and wholebody images were taken with a Leica MZ16F fluorescence stereomicroscope. The number of neutrophils (*mpx*⁺ or *lyz*⁺)*,* macrophages (*mfap4*⁺)*,* erythrocytes (*gatala*⁺, in the yolk sac extension) and HSPCs (*runx1*⁺) was determined by counting them visually in blinded samples and fluorescence.

### Example 1.5. Primary cell collection and culture

Bone marrow aspirates were collected at the Hospital General Universitario José Maria Morales Meseguer under CEIC approval number EST: 12/16. BMMCs were obtained by centrifugation using Histopaque-1077 (Sigma-Aldrich). Human HSC colony assays were performed in human methylcellulose complete medium (#HSC003, R&D Systems) following the manufacturer's instructions. BMMCs were incubated in methylcellulose medium at 37 °C in a 5% CO₂-humidified atmosphere in the presence of DMSO or nilotinib (0.01 and 0.1 µM), and colonies were counted at day 9 and 14 using standard morphological criteria.

Human cord blood CD34⁺/CD133⁺ HSPCs were purchased from ZenBio (#SER-CD34-F), expanded for 4 d in StemSpan SFEM (#09650) and CC100 (#02690) (both from Stem Cell Technologies) and then differentiated in erythroid differentiation medium [IMDM with stabilized glutamine (ThermoFisher Scientific, #12440-061), 2% human AB plasma (SeraCare, #501973), 3% human AB serum (Atlanta Biologicals, #S40110), 1% Pen/Strep (ThermoFisher Scientific), 3UI/ml heparin (Sigma-Aldrich, #H3149), 10 µg/ml insulin (Sigma-Aldrich, #I9278-5ML), 200 µg/ml holo-transferrin (Sigma-Aldrich, #T0665), 1 UI EPO (Peprotech, # 100-64), 10 ng/ml SCF (PeproTech, #300-07), 1 ng/ml IL-3 (PeproTech, #200-03)] in the presence or absence of 0.1 µM nilotinib for 10 days. Cells were stained with anti-CD235a-APC (#17-9987-41) and anti-CD71-FITC (#11-0719-41) (both from ThermoFisher Scientific), and analyzed by flow cytometry.

### Example 1.6. Erythroid differentiation assay

K562 cells (CRL-3343; American Type Culture Collection) were maintained in RPMI culture medium supplemented with 10% fetal calf serum (FCS), 2 mM Glutamine, and 1% penicillin-streptomycin (Life Technologies). Cells were maintained and subcultured before confluence every 72 h. For differentiation, cells were treated with 50 µM hemin (#16009-13-5, Sigma-Aldrich) in the presence of 0.1% DMSO alone or with the caspase-1 inhibitor VX-765 (100 µM), the tyrosine kinase inhibitor nilotinib (0.1 µM), the RNA polymerase I inhibitor CX-5461 (200 nM, 1 µM and 10 µM) (all from MedChemExpress). Cells were collected at different time points (0, 24, 48 hours post-hemin addition), centrifuged, washed twice with PBS, and stored at -80 °C for further analysis.

### Example 1.7. Immunoblotting and immunoprecipitation

Cells were lysed in 50 mM Tris-HCl (pH 7.5), 150 mM NaCl, 1% (w/v) NP-40 and fresh protease inhibitor (1/20, #P8340, Sigma-Aldrich). Protein quantification was performed with the BCA kit using BSA as standard. Cell lysates (40 µg) in SDS sample buffer were subjected to electrophoresis on a polyacrylamide gel and transferred to nitrocellulose membranes. The membranes were incubated for 1 h with TTBS containing 5% (w/v) skimmed dried milk powder or 2% (w/v) BSA. The membranes were immunoblotted in the same buffer 16 h at 4°C with the different primary antibodies diluted 1/1000. The blots were then washed with TTBS and incubated for 1 h at room temperature with secondary HRP-conjugated antibodies diluted 2,500-fold in 5% (w/v) skimmed milk in TTBS. After repeated washes, the signal was detected with enhanced chemiluminescence reagent and ChemiDoc XRS Biorad.

PhosTag SDS-PAGE was used to analyze the phosphorylation of ZAKα. Briefly, 30 µM Phos-tag Acrylamide (Wako Chemicals, AAL-107) and 60 µM MnCl₂ were added to homemade 10% SDS-PAGE gel. PhosTag-SDS-Agarose-PAGE gels were made to 3% polyacrylamide and 0.5% agarose with a final concentration of Phos-tag Acrylamide and MnCl2 as mentioned above. Cells were directly harvested using Laemmli buffer, lysed with an ultrasonicator, and loaded into the Phos-tag gel to run. Once the run was completed, the polyacrylamide gel was washed in transfer buffer with 10 mM EDTA twice, subsequently washed once without EDTA, blotted onto 0.45 µm PVDF membranes (Bio-rad), blocked with 3% milk, and incubated with primary and corresponding secondary antibodies.

The primary antibodies used were rabbit human GATA1 (#3535, Cell Signaling), human NLRP1 (#AF6788, R&D Systems and #67980, Biolegend), human ZAKα (#A301-993A, Bethyl Laboratories), human phosphoP38 (#MA5-15177, ThermoFisher Scientific), ACTB-HRP (#sc-47778, Santa Cruz Biotechnology), and ANTI-FLAG_M2-HRP. The secondary antibodies used were anti-sheep (#31480, Thermofisher), anti-rabbit (#A6154, Sigma-Aldrich), and anti-mouse (#A4416, Sigma-Aldrich) Igs.

### Example 1.8. Statistical analysis

Data are shown as mean ± SEM and were analyzed by analysis of variance (ANOVA) and a Tukey or Bonferroni multiple range test to determine differences among groups. Differences between two samples were analyzed by Student t-test.

### Example 2. Results

### Example 2.1. ZAKα activates the NLRP1 inflammasome in HSPCs after ribosomal stress in zebrafish and human

Given that recent evidence indicates that cellular stress, including oxidative nucleic acid damage and ribotoxic stress, activates ZAKα/P38 axis that phosphorylates and activates human NLRP1 inflammasome independently of DPP9 in keratinocytes, the inventors tested whether a similar mechanism operates to regulate hematopoiesis. In K562 cells, nilotinib - a kinase inhibitor that has a high affinity for ZAKα - strongly promoted erythroid differentiation of K562, even in the absence of hemin **(****Figure 1A****).** Notably, erythroid differentiation with hemin resulted in phosphorylation of P38 and ZAKα, and degradation of GATA1, whereas nilotinib strongly reversed all of them **(****Figures 1A** **and** **2****).** In addition, nilotinib was also able to promote accumulation of GATA1 in the presence of CX-5461 **(****Figure 1C****),** an RNA polymerase I inhibitor that impairs erythroid differentiation in K562 cells **(****Figures 1D****)** and is widely used to model Diamond-Blackfan anemia (DBA). Similarly, treatment of zebrafish larvae with nilotinib caused a decrease in neutrophil number **(****Figures 3A**, **3B**) in wild type larvae and reduced neutrophilia in the Spint1a-deficient model of neutrophilic inflammation **(****Figures 3C**, **3D**), whereas the antibiotic anisomycin, which promotes ribotoxic stress-mediated activation of NLRP1, caused neutrophilia **(****Figures 3A**, **3B**).

We next investigated whether the effects of nilotinib and anisomycin in hematopoiesis were mediated through ZAKα. Interestingly, although human ZAKα and ZAKβ are generated by alternative splicing from the same gene and only ZAKα can interact with the ribosome, zebrafish showed two genes: *map3k20a* encoding Zaka and *map3k20b* encoding Zakb **(****Figure 4A****).** Although knockdown of Zaka and Zakb (60 and 80% editing efficiency, respectively) did not result in any obvious developmental defects **(****Figures 4B-4E****),** Zaka deficiency phenocopied the effects of Nlrp1 deficiency in zebrafish larvae; that is, increased erythrocyte numbers **(****Figures 3E and 3F****),** reduced neutrophil **(****Figure 3G, 3H****,** **5C** **and** **5D****)** and macrophage counts (**Figures 3I and 3J**) and decreased caspase-1 activity **(****Figure 3K****).** In sharp contrast, Zakb deficiency did not affect neutrophil numbers **(****Figures 5A**, **5B**). Importantly, anisomycin failed to increase neutrophil number in Nlrp1- and Zaka-deficient larvae **(****Figures 5C**, **5D**), suggesting that Zaka acts upstream of Nlrp1 activation.

The observation that the activation of NLRP1 by ZAKα following cellular stress was conserved in zebrafish and human was unexpected, as mouse NLRP1s are not activated by ribotoxic stress and both mouse and zebrafish lack a C-terminal PYD domain. However, the linker domain of zebrafish Nlrp1 showed a relatively well conserved serine and threonine residues, including S107, which has been shown to be directly phosphorylated by P38 and to be important for human NLRP1 activation by this mechanism. To confirm the conservation of this activation mechanism in zebrafish and gain further insight into the relevance of NLRP1 phosphorylation by ZAKα, we expressed human wild type S107A and S107D NLRP1 in zebrafish larvae and found that both wild type and S107D, but not S107A, increased neutrophil number (**Figures 6A-6C**) and caspase-1 activity **(****Figure 6D****).** Strikingly, although nilotinib was able to abrogate neutrophilia and caspase-1 activation induced by wild type NLRP1, it failed to reverse neutrophilia and caspase-1 activation induced by NLRP1 with phosphomimetic S107D mutation **(****Figures 6B-D** and **7**). These results confirmed that the activation of NLRP1 inflammasome is conserved in zebrafish and human, and that NLRP1 is activated by phosphorylation of the linker domain following activation of ZAKα in HSPCs to regulate the erythroid-myeloid lineage decision.

### Example 2.2. Nilotinib promotes erythropoiesis in bone marrow mononuclear cells from a DBA patient and erythroid differentiation of CD34⁺/CD133⁺ HSPCs from healthy donors

These findings led the inventors to study the relevance of this signaling pathway in DBA, a pathology in which reduced ribosome levels selectively impair translation of a subset of mRNAs, including *GATA1* mRNA, and thus, activation of the NLRP1 inflammasome by ZAKα is also expected. We found that nilotinib strongly increased the number of erythroid colonies derived from bone marrow mononuclear cells of a DBA patient with a *RPS19* mutation, a mutation that is associated to DBA **(****Figure 6E****).** Importantly, the number of myeloid colonies was not affected by nilotinib **(****Figure 6F****),** suggesting that inhibition of NLRP1 inflammasome activation may alleviate impaired erythropoiesis in DBA. In addition, nilotinib also accelerated erythropoiesis of primary CD34⁺/CD133⁺ HSPCs from healthy donors **(****Figures 8** **and** **9****).**

### Example 2.3. Imatinib and dasatinib phenocopy the effects of nilotinib in human K562 cells and zebrafish larvae

The inventors then assessed the effects of imatinib and dasatinib, two tyrosine kinase inhibitors approved by the FDA/EMA with the same indications as nilotinib. Strikingly, both imatinib and dasatinib phenocopied the effects of nilotinib in both human K562 cells and zebrafish larvae. They promoted erythroid differentiation of K562 cells by blocking the ZAKα/P38 axis **(****Figure 10A****)** and enhanced the number of erythrocytes in zebrafish larvae **(****Figure 10B****).**

## Claims

1. ZAK alpha kinase inhibitors for use in the treatment of congenital anemia or anemia of inflammation.

2. ZAK alpha kinase inhibitors for use, according to claim 1, wherein the congenital anemia is selected from the group comprising: Diamond-Blackfan anemia, Fanconi anemia and/or thalassemia.

3. ZAK alpha kinase inhibitors for use, according to any of the previous claims, wherein the anemia of inflammation is a non-ferropenic acquired anemia.

4. ZAK alpha kinase inhibitors for use, according to any of the previous claims, wherein the inhibitor is a tyrosine kinase inhibitor.

5. ZAK alpha kinase inhibitors for use, according to any of the previous claims, wherein the inhibitor is a tyrosine kinase inhibitor selected from the group comprising: Nilotinib, Dasatibin, Imatinib, Ponatinib and/or Bosutinib.

6. ZAK alpha kinase inhibitors selected from the group comprising: Nilotinib, Dasatibin, Imatinib, Ponatinib and/or Bosutinib, for use, according to any of the previous claims, in the treatment of congenital anemia.

7. Composition comprising ZAK alpha kinase inhibitors and, optionally, pharmaceutically acceptable excipients or carriers, for use in the treatment of congenital anemia or anemia of inflammation.

8. Composition comprising ZAK alpha kinase inhibitors for use, according to claim 7, wherein the congenital anemia is selected from the group comprising: Diamond-Blackfan anemia, Fanconi anemia and/or y thalassemia.

9. Composition comprising ZAK alpha kinase inhibitors for use, according to any of the claims 7 or 8, wherein the anemia of inflammation is a non-ferropenic acquired anemia.

10. Composition comprising ZAK alpha kinase inhibitors for use, according to any of the claims 7 to 9, wherein the inhibitor is a tyrosine kinase inhibitor.

11. Composition comprising ZAK alpha kinase inhibitors for use, according to any of the claims 7 to 10, wherein the inhibitor is a tyrosine kinase inhibitor selected from the group comprising: Nilotinib, Dasatibin, Imatinib, Ponatinib and/or Bosutinib.

12. Composition comprising ZAK alpha kinase inhibitors selected from the group comprising: Nilotinib, Dasatibin, Imatinib, Ponatinib and/or Bosutinib, for use, according to any of the claims 7 to 11, in the treatment of congenital anemia.

13. *In vitro* method for screening, identifying and/or producing compounds for use in the treatment of anemia which comprises: a) Assessing ZAK alpha kinase enzyme activity once the candidate compound has been incubated with ZAK alpha kinase enzyme, and b) wherein if an inhibition of ZAK alpha kinase enzyme activity is observed, it is indicative that the candidate compound may be effective in the treatment of anemia.
